# FASCICULE DE BREVET EUROPEEN

(11) **EP 0 725 056 B1**
(45) Date de publication et mention de la délivrance du brevet: **15.01.1997**
(21) Numéro de dépôt: 95119956.1
(22) Date de dépôt: 18.12.1995
(51) Int. Cl.: C07C 271/22, C07C 311/06, A61K 7/021, A61K 7/027, A61K 7/031, A61K 7/032, A61K 7/035, A61K 7/043, A61K 7/16, A61K 7/48

(54) **Dérivés perfluoroalkyle de lysine, procédé de préparation, leur utilisation notamment en cosmétique et compositions les comprenant**
Perfluoroalkylierte Lysinderivate, Verfahren zu ihrer Herstellung, ihre Verwendung insbesondere in der Kosmetik und diese enthaltende Zusammensetzungen
Perfluoroalkylated derivatives of lysine, process for their preparation, their use especially in cosmetics and compositions containing them

(30) Priorité: 31.01.1995 FR 9501112
(43) Date de publication de la demande: 07.08.1996
(73) Titulaire: L'OREAL, 75008 Paris (FR)
(72) Inventeur: Bordier, Thierry, F-93290 Tremblay en France (FR); Philippe, Michel, F-91320 Wissous (FR)
(74) Mandataire: Dodin, Catherine

(56) Documents cités:
- EP-A- 0 139 481
- EP-A- 0 336 265
- EP-A- 0 447 287
- EP-A- 0 545 786

## Description

La présente invention a pour objet des composés à groupement fluoroalkyloxycarbonyle ou fluoroalkylsulfonyle, dérivés de la lysine, leur procédé de préparation, leur utilisation notamment en cosmétique et les compositions, notamment cosmétiques, les comprenant.

On connaît des compositions, notamment cosmétiques, pharmaceutiques ou alimentaires, qui peuvent se présenter sous forme d'une poudre dite compacte, obtenue par compactage. Il s'agit généralement de compositions anhydres pouvant être constituées principalement de particules solides et d'un liant gras, mises en forme par compression.
L'élaboration de telles compositions soulève toutefois de nombreuses difficultés car la composition finale doit être suffisamment homogène et compacte pour présenter une bonne aptitude au prélèvement et pour éviter par ailleurs une fragmentation pouvant être provoquée notamment par des chocs.

Il est décrit, dans la demande de brevet EP139481, des compositions cosmétiques utilisant comme agents pour modifier la surface de composés inorganiques, en vue d'en augmenter la dispersibilité, soit un dérivé monoacylé d'un acide aminé basique dont le groupement acyle aliphatique a 8-22 atomes de carbone, soit un dérivé N,N-diacylé d'un acide aminé basique dont les groupements acyles identiques ou différents, ont 1-22 atomes de carbone.
Il est également décrit, dans la demande de brevet EP336265, des compositions cosmétiques pour la mise en forme des cheveux comprenant comme agents tensioactifs, un dérivé N-monoacylé d'un acide aminé basique dont le groupement acyle a 8-22 atomes de carbone.
On constate toutefois que les dérivés acylés des acides aminés basiques décrits précédemment sont très difficilement compactables, voire intassables.

L'invention a pour but de proposer de nouveaux composés, qui permettent de faciliter l'obtention de telles compositions, tout en satisfaisant aux exigences précitées, sans présenter les inconvénients de l'art antérieur.

La présente invention a donc pour objet un dérivé de la lysine à groupements N^{ε}-fluoroalkyloxycarbonyle ou N^{ε}-fluoroalkylsulfonyle de formule (I)

HOOC - CH (NH₂) - (CH₂)₄ - NH - X - (CH₂)ₙ - R

dans laquelle n est compris entre 0 et 4, X représente un groupement divalent COO ou SO₂ et R représente un radical perfluoroalkyle, linéaire ou ramifié, ayant 4 à 20 atomes de carbone,
les sels des composés de formule (I), leurs isomères optiques de configuration D ou L, et leurs mélanges.

Un autre objet de l'invention est une composition, entre autre cosmétique, pharmaceutique, hygiénique ou alimentaire, comprenant au moins un dérivé de formule (I).
Encore un objet de l'invention est l'utilisation d'au moins un dérivé de formule (I) comme substance de revêtement de particules substrat. On a en effet constaté que lesdites particules, généralement des poudres, une fois compactées, permettent une bonne cohésion du produit, d'où un produit compacté qui ne s'effrite pas facilement.

On a de plus constaté que les dérivés selon l'invention permettent également de conférer à la composition cosmétique les comprenant des qualités d'étalement, d'adhésion à la peau et de diffusion de la lumière particulièrement intéressantes, ainsi qu'un toucher onctueux et agréable et une meilleure résistance à l'eau, dûs probablement à la présence de la chaîne fluorée.

Plus précisément, l'invention a pour objet un dérivé de la lysine de formule (I) :

HOOC - CH (NH₂) - (CH₂)₄ - NH - X - (CH₂)ₙ - R

dans laquelle n est compris entre 0 et 4, X représente un groupement divalent COO ou SO₂ et R représente un radical perfluoroalkyle, linéaire ou ramifié, ayant 4 à 20 atomes de carbone,
les sels des composés de formule (I), leurs isomères optiques de configuration D ou L, et leurs mélanges.

Par radical perfluoroalkyle, on entend un radical dont tous les atomes d'hydrogène sont remplacés par des atomes de fluor.
Le radical R peut de préférence représenter un radical alkyle, linéaire ou ramifié, ayant de 4 à 8 atomes de carbone.
Parmi les dérivés selon l'invention, on peut citer la N^{ε}-2-F-octyl-éthyloxycarbonyl-L-lysine et la N^{ε}-2-F-hexyl-éthyloxycarbonyl-L-lysine.

Les sels des dérivés de formule (I) peuvent être choisis parmi les sels de cations inorganiques monovalents, tels que ceux de sodium, ou divalents tels que ceux de zinc ou de cuivre. Les sels peuvent être aussi choisis parmi les sels de cations organiques tels que ceux d'aminopropanediol, de trishydroxyaminométhane, de glucamine et de N-méthylglucamine.

Les dérivés selon l'invention peuvent se présenter sous forme solide ayant une taille des particules comprise entre 10 - 500 000 nm, de préférence comprise entre 100 - 25000 nm.

Ces dérivés sont généralement insolubles dans les huiles et dans les solutions aqueuses dont le pH est compris entre 5 et 8. Ils présentent généralement un point de fusion élevé, supérieur à 200°C.

La composition selon l'invention comprenant lesdits dérivés peut se présenter sous diverses formes telles que des dispersions, des lotions éventuellement épaissies ou gélifiées, des poudres éventuellement compactées, des laits, des crèmes, des sticks, des mousses ou des sprays lorsqu'elle est conditionnée en aérosol, des émulsions huile-dans-eau ou eau-dans-huile, de dispersions liposomiales ou encore de préparations solides. Les dérivés selon l'invention peuvent être compris dans la composition en une proportion allant de 0,05% à 80% en poids, de préférence en une proportion de 0,5 à 30% en poids par rapport au poids total de la composition.
Les dérivés selon l'invention peuvent être présents dans la composition sous forme libre et/ou sous forme d'une association avec des particules substrats qu'ils enrobent.

Outre le dérivé selon l'invention, la composition peut aussi comprendre au moins un additif choisi dans le groupe constitué par les agents tensioactifs, les corps gras, les solvants organiques, les silicones, les épaississants, les adoucissants, les filtres solaires, les agents de traitement, les agents anti-mousse, les agents hydratants, les parfums, les conservateurs, les agents antioxydants, les séquestrants, les agents de sapidité, les agents alcalinisants ou acidifiants, les charges et les pigments.
Parmi les corps gras utilisables dans la composition selon l'invention, on peut citer les huiles, les cires, les acides gras, les alcools gras et/ou leur mélange.
Les huiles et les cires peuvent être d'origine animale, végétale, minérale ou de synthèse. On peut citer en particulier l'huile de palme hydrogénée, l'huile de ricin hydrogénée, l'huile de vaseline, l'huile de paraffine, l'huile de purcellin. On peut également citer la cire d'abeille, la cire de montan, la cire de Carnauba, la cire de candelilla, la cire de canne à sucre, la cire du Japon, l'ozokérite, les cires microcristallines, la cire de paraffine, la cire de lanoline, la cire de lanoline hydrogénée et la cire de lanoline acétylée.

Parmi les particules pouvant être enrobées par les dérivés selon l'invention, on peut citer notamment les pigments, les charges particulaires et les microsphères telles que les microsphères creuses de copolymères de chlorure de vinylidène/acrylonitrile. Plus particulièrement, on peut citer, parmi les charges, les charges insolubles éventuellement colorées telles que des nanopigments d'oxydes métalliques comme les oxydes de titane, de zinc, de fer, de manganèse, de césium et/ou de zirconium.

La composition selon l'invention peut se présenter sous forme de compositions de maquillage telles que des fonds de teint, des crèmes teintées, des mascaras, des fards à joues et à paupières, des rouges à lèvres, des vernis à ongles.
Selon une forme particulière de réalisation, elle peut se présenter sous forme dite compacte, le dérivé selon l'invention facilitant le compactage des ingrédients desdites compositions et notamment la cohésion du produit compacté. Parmi ces compositions compactes, on peut citer notamment les fonds de teint, les fards à joues ou à paupières, et les rouges à lèvres.

La composition selon l'invention peut également se présenter sous forme d'une composition pharmaceutique ou hygiénique telles que des pâtes dentifrices, des compositions exfoliantes, des poudres pour le corps ou pour bébés et des poudres antitranspirantes, voire sous forme d'une composition alimentaire.

L'invention a également pour objet un procédé de préparation de dérivé de formule (I), consistant :
- à faire réagir en milieu aqueux et à pH basique, de la lysine ou l'un de ses sels, de configuration connue, avec une solution d'un sel de cuivre,
- à faire réagir sur la solution du complexe de cuivre un composé de formule (II) :

   R - (CH₂)ₙ - X - Y

   dans laquelle n est compris entre 0 et 4, R et X sont tels que définis précédemment et Y est choisi dans le groupe constitué par un atome de chlore, un radical chlorométhyle et un radical imidazolyle, ledit composé de formule (II) étant additionné avec ou sans solvant,
- à traiter le sel de cuivre de la lysine substituée obtenu par un agent décomplexant et, éventuellement
- à purifier le composé obtenu.
Parmi les solutions de sels de cuivre utilisées dans le procédé selon l'invention, on peut citer en particulier les solutions de sulfate de cuivre.
Le pH basique du milieu réactionnel, est de préférence compris entre 8 et 14.
Selon un mode de réalisation préférée du procédé de préparation, l'agent décomplexant utilisé est une solution aqueuse du sel disodique de l'acide éthylènediamine-tétracétique.

On va maintenant donner à titre illustratif des exemples de préparation de dérivés de la lysine selon l'invention ainsi qu'un exemple de composition comprenant un tel dérivé.

Les exemples de préparation sont obtenus selon le mode opératoire qui suit :
Dans un tricol de 250 ml muni d'un thermomètre et d'une ampoule à introduction de 50 ml, 10 g de L-lysine monochlorhydrate sont dissous dans 44 ml d'une solution aqueuse de soude à 10%.
Une solution de 6,8 g de sulfate de cuivre pentahydrate dans 40 ml d'eau est introduite dans le milieu réactionnel.
Au mélange homogène refroidi à la température de 5°C, 4,6 g d'hydrogénocarbonate de sodium sont ajoutés suivi par l'addition goutte à goutte de chloroformiate d'alkyle fluoré.
Après une nuit sous agitation à température ambiante, le milieu réactionnel est filtré et le précipité correspondant au produit final sous forme de complexe de cuivre, est lavé à l'eau et à l'acétone, puis séché à l'étuve sous pression réduite.
Pour éliminer le cuivre, x moles de complexes sont traitées au reflux pendant 4 heures par une solution aqueuse de sel disodique dihydraté de l'acide éthylènediamine-tétraacétique à 10%. Le traitement peut être reconduit plusieurs fois pour obtenir un produit totalement décomplexé.

### EXEMPLE 1 : Préparation de la N^{ε}-2-perfluorooctyl-éthyloxycarbonyl-L-Lysine

En suivant le mode opératoire précédent, on obtient 27 g (rendement de 77%) de produit blanc, en utilisant le chloroformiate d'heptadécafluorodécanol en tant que chloroformiate d'alkyle fluoré.

L'analyse chimique donne les caractéristiques suivantes :
- Fusion : T>260°C (mesurée au bankofler)
- Spectre de masse : m/z 637 (MH)+
- Analyse élémentaire :(C17H17F17N2O4, poids moléculaire : 636,309)

| | C | H | F | N |
|---|---|---|---|---|
| % calculé | 32,09 | 2,69 | 50,76 | 4,40 |
| % mesuré | 31,27 | 2,52 | 48,93 | 3,79 |

- Granulométrie (mesurée au moyen d'un Coulter Counter TA2) : en nombre (moyenne) : 2,80 µm (écart type : 2,32 µm)
- Pouvoir rotatoire : [α]_{D} = + 4° concentration c= 1, solvant :CH3COOH

### EXEMPLE 2 : Préparation de la N^{ε}-2-perfluorohexyl-éthyloxycarbonyl-L-Lysine

En suivant le mode opératoire précédent, on obtient 23 g d'un produit blanc (rendement de 77%) en utilisant le chloroformiate de tridécafluorooctanol.
L'analyse chimique donne les caractéristiques chimiques suivantes :
- Fusion : T>260°C (mesurée au bankofler)
- Spectre de masse : m/z = 537 (MH)+
- Analyse élémentaire : (C15H17F13N2O4 ; poids moléculaire 536,293)

| | C | H | F | N | Cu |
|---|---|---|---|---|---|
| % calculé | 33,59 | 3,20 | 46,05 | 5,22 | |
| % mesurée | 32,67 | 2,99 | 45,05 | 4,53 | 67 ppm |

- Granulométrie : (mesurée au moyen d'un Coulter Counter TA2) : en nombre (moyenne) : 3,21 µm (écart type : 2,95 µm)
- Pouvoir rotatoire : [α]_{D} = + 4° concentration c= 1, solvant :CH3COOH

### EXEMPLE 3

On prépare une poudre compactée ayant la composition suivante :

| *Composition A :* | |
|---|---|
| - Talc | 38,4 g |
| - Oxychlorure de bismuth | 10 g |
| - Stéarate de zinc | 4 g |
| - Composé de l'exemple 1 | 20 g |
| - Poudre de Nylon | 20 g |

| *Composition B :* | |
|---|---|
| - Oxydes de fer | 1,6 g |
| - Huile de vaseline | 6 g |

La poudre est obtenue de la façon suivante : on broie la composition A dans un broyeur de type KENWOOD pendant environ 5 minutes sous faible agitation. On ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles.

On obtient une poudre compactée présentant une bonne adhésion, qui s'étale bien et de manière agréable sur la peau, tout en étant douce au toucher. De plus, la présence du composé de l'exemple 1 évite un effritement trop aisé du produit compacté.

## Revendications

1. Dérivé de la lysine de formule (I)
HOOC - CH (NH₂) - (CH₂)₄ - NH - X - (CH₂)ₙ - R
dans laquelle n est compris entre 0 et 4, X représente un groupement divalent COO ou SO^{₂}, et R représente un radical perfluoroalkyle, linéaire ou ramifié, ayant 4 à 20 atomes de carbone,
les sels des composés de formule (I), leurs isomères optiques de configuration D ou L, et leurs mélanges.

2. Dérivé selon la revendication 1, caractérisé en ce que les sels sont choisis parmi les sels de cations inorganiques monovalents ou divalents et les sels de cations organiques.

3. Dérivé selon l'une des revendications précédentes, caractérisé en ce que le radical R représente un radical perfluoroalkyle, linéaire ou ramifié, ayant de 4 à 8 atomes de carbone.

4. Dérivé selon l'une des revendications précédentes, caractérisé en ce qu'il est choisi parmi la N^{ε}-2-perfluorooctyl-éthyloxycarbonyl-L-lysine et la N^{ε}-2-perfluorohexyl-éthyloxycarbonyl-L-lysine.

5. Dérivé selon l'une des revendications précédentes, caractérisé en ce qu'il a une taille de particules comprise entre 10 nm et 500 000 nm.

6. Composition caractérisée en ce qu'elle comprend au moins un dérivé selon l'une des revendications 1 à 5.

7. Composition selon la revendication 6, caractérisée en ce que le dérivé de la lysine est compris entre 0,05% et 80% en poids par rapport au poids total de la composition.

8. Composition selon la revendication 7, caractérisée en ce que le dérivé de la lysine est compris en une proportion de 0,5 à 30% en poids par rapport au poids total de la composition.

9. Composition selon l'une des revendications 6 à 8, caractérisée en ce qu'elle comprend le dérivé de lysine sous forme libre et/ou sous forme d'une association avec des particules substrats qu'il enrobe.

10. Composition selon la revendication 9, dans laquelle les particules substrats sont choisies parmi les pigments, les charges particulaires et les microsphères telles que les microsphères creuses de copolymères de chlorure de vinylidène/acrylonitrile.

11. Composition selon l'une des revendications 6 à 10, se présentant sous forme d'une composition cosmétique, pharmaceutique, hygiénique ou alimentaire.

12. Composition selon l'une des revendications 6 à 11, se présentant sous forme d'un fond de teint, mascara, fard à joues et à paupières, rouge à lèvres, vernis à ongles, composition exfoliante, pâte dentifrice, poudre pour le corps ou pour bébés et/ou poudre antitranspirante.

13. Procédé de préparation d'un dérivé selon l'une des revendications 1 à 5, caractérisé en ce qu'il consiste à faire réagir en milieu aqueux et à pH basique, de la lysine ou l'un de ses sels, de configuration connue, avec une solution d'un sel de cuivre, puis à faire réagir sur la solution du complexe de cuivre un composé de formule R - (CH₂)ₙ - X - Y
dans laquelle n est compris entre 0 et 4, R et X sont tels que définis précédemment, et Y est choisi dans le groupe constitué par un atome de chlore, un radical chlorométhyle et un radical imidazolyle, puis à traiter le sel de cuivre obtenu par un agent décomplexant.

14. Utilisation d'au moins un dérivé selon l'une des revendication 1 à 5, en tant que revêtement de particules substrat.

15. Utilisation d'au moins un dérivé selon l'une des revendications 1 à 5, pour faciliter le compactage de compositions comprenant des particules.

## Claims

1. Lysine derivative of formula (I)
HOOC-CH(NH₂)-(CH₂)₄-NH-X-(CH₂)ₙ-R
in which n is between 0 and 4, X represents a divalent COO or SO₂ group and R represents a linear or branched perfluoroalkyl radical having 4 to 20 carbon atoms,
the salts of the compounds of formula (I), their optical isomers of D or L configuration, and their mixtures.

2. Derivative according to Claim 1, characterized in that the salts are chosen from the salts of monovalent or divalent inorganic cations and the salts of organic cations.

3. Derivative according to one of the preceding claims, characterized in that the R radical represents a linear or branched perfluoroalkyl radical having from 4 to 8 carbon atoms.

4. Derivative according to one of the preceding claims, characterized in that it is chosen from N^{ε}-2-(perfluorooctyl)ethyloxycarbonyl-L-lysine and N^{ε}-2-(perfluorohexyl)ethyloxycarbonyl-L-lysine.

5. Derivative according to one of the preceding claims, characterized in that it has a particle size of between 10 nm and 500,000 nm.

6. Composition, characterized in that it comprises at least one derivative according to one of Claims 1 to 5.

7. Composition according to Claim 6, characterized in that the lysine derivative is between 0.05 % and 80 % by weight with respect to the total weight of the composition.

8. Composition according to Claim 7, characterized in that the lysine derivative is included in a proportion from 0.5 to 30 % by weight with respect to the total weight of the composition.

9. Composition according to one of Claims 6 to 8, characterized in that it comprises the lysine derivative in the free form and/or in the form of a combination with substrate particles which it coats.

10. Composition according to Claim 9, in which the substrate particles are chosen from pigments, particulate fillers and microspheres such as hollow vinylidene chloride/acrylonitrile copolymer microspheres.

11. Composition according to one of Claims 6 to 10, which is provided in the form of a cosmetic, pharmaceutical, hygiene or food composition.

12. Composition according to one of Claims 6 to 11, which is provided in the form of a foundation, mascara, blusher, eye shadow, lipstick, nail varnish, exfoliative composition, toothpaste, powder for the body or for babies, and/or anti-perspirant powder.

13. Process for the preparation of a derivative according to one of Claims 1 to 5, characterized in that it consists in reacting, in aqueous medium and at basic pH, lysine or one of its salts, of known configuration, with a solution of a copper salt, in then reacting the solution of the copper complex with a compound of formula
R-(CH₂)ₙ-X-Y
in which n is between 0 and 4, R and X are as defined above and Y is chosen from the group consisting of a chlorine atom, a chloromethyl radical and an imidazolyl radical, and in then treating the copper salt obtained with a decomplexing agent.

14. Use of at least one derivative according to one of Claims 1 to 5, as coating for substrate particles.

15. Use of at least one derivative according to one of Claims 1 to 5, for facilitating compaction of compositions comprising particles.

## Patentansprüche

1. Lysinderivate der Formel (I)
HOOC-CH(NH₂)-(CH₂)₄-NH-X-(CH₂)ₙ-R (I)
worin n im Bereich von 0 bis 4 liegt, X eine zweiwertige COO- oder SO₂- Gruppe bedeutet und R eine geradkettige oder verzweigte Perfluoralkylgruppe mit 4 bis 20 Kohlenstoffatomen ist,
die Salze der Verbindungen der Formel (I), ihre optischen Isomere in D- oder L-Konfiguration und deren Gemische.

2. Derivate nach Anspruch 1, dadurch gekennzeichnet, daß die Salze unter Salzen von einwertigen oder zweiwertigen anorganischen Kationen und Salzen von organischen Kationen ausgewählt sind.

3. Derivate nach einem der vorhergehenden Ansprüche, dadurch gekennzeichnet, daß die Gruppe R eine lineare oder verzweigte Perfluoralkylgruppe mit 4 bis 8 Kohlenstoffatomen bedeutet.

4. Derivate nach einem der vorhergehenden Ansprüche, dadurch gekennzeichnet, daß sie unter N^{ε}-2-Perfluoroctyl-ethyloxycarbonyl-L-lysin und N^{ε}-2-Perfluorhexylethyloxy-carbonyl-L-lysin ausgewählt sind.

5. Derivate nach einer der vorhergehenden Ansprüche, dadurch gekennzeichnet, daß sie eine Partikelgröße im Bereich von 10 nm bis 500000 nm aufweisen.

6. Zusammensetzung, dadurch gekennzeichnet, daß sie mindestens ein Derivat nach einem der Ansprüche 1 bis 5 enthält.

7. Zusammensetzung nach Anspruch 6, dadurch gekennzeichnet, daß das Lysinderivat in Anteilen von 0,05 bis 80 Gew.-%, bezogen auf das Gesamtgewicht der Zusammensetzung, enthalten ist.

8. Zusammensetzung nach Anspruch 7, dadurch gekennzeichnet, daß das Lysinderivat in Anteilen von 0,5 bis 30 Gew.-%, bezogen auf das Gesamtgewicht der Zusammensetzung, enthalten ist.

9. Zusammensetzung nach einem der Ansprüche 6 bis 8, dadurch gekennzeichnet, daß sie das Lysinderivat in freier Form und/oder in Form einer Kombination mit Substratpartikeln, die es umhüllt, enthält.

10. Zusammensetzung nach Anspruch 9, in der die Substrat-partikel unter Pigmenten, speziellen Füllstoffen und Mikrokügelchen ausgewählt sind, wie hohlen Mikrokügelchen von Vinylidenchlorid-Acrylnitril-Copolymeren.

11. Zusammensetzung nach einem der Ansprüche 6 bis 10, die in Form einer kosmetischen, pharmazeutischen Zusammensetzung oder Zusammensetzung zur Hygiene oder einer Lebensmittelzusammensetzung vorliegt.

12. Zusammensetzung nach einem der Ansprüche 6 bis 11, die als Make up, Mascara, Wangenrouge, Lidschatten, Lippenstift, Nagellack, Peelingzusammensetzung, Zahnpasta, Körperpuder oder Babypuder und/oder Antitranspirantpuder vorliegt.

13. Verfahren zur Herstellung eines Derivats nach einem der Ansprüche 1 bis 5, dadurch gekennzeichnet, daß es darin besteht, Lysin oder eines seiner Salze bekannter Konfiguration mit einer Kupfersalzlösung und anschließend die Lösung des Kupferkomplexes mit einer Verbindung der Formel R-(CH₂)ₙ-X-Y,
worin n im Bereich von 0 bis 4 liegt, R und X die oben angegebenen Bedeutungen aufweisen und Y unter einem Chloratom, einer Chlormethylgruppe und einer Imidazolylgruppe ausgewählt ist, in wäßrigem Medium bei einem basischen pH-Wert umzusetzen, und dann das erhaltene Kupfersalz mit einem Dekomplexierungsmittel zu behandeln.

14. Verwendung mindestens eines Derivats nach einem der Ansprüche 1 bis 5 als Überzug von Substratpartikeln.

15. Verwendung mindestens eines Derivats nach einem der Ansprüche 1 bis 5 zu Erleichterung der Verdichtung von Zusammensetzungen, die Partikel enthalten.
